# EUROPÄISCHE PATENTSCHRIFT

(11) **EP 1 716 234 B1**
(45) Veröffentlichungstag und Bekanntmachung des Hinweises auf die Patenterteilung: **02.10.2013**
(21) Anmeldenummer: 04738565.3
(22) Anmeldetag: 19.05.2004
(51) Int. Cl.: C12N 15/117, A61K 31/713, A61K 47/48, A61K 39/39, A61K 48/00

(54) **SUBSTITUIERTES, NICHT-KODIERENDES NUKLEINSÄUREMOLEKÜL ZUR THERAPEUTISCHEN UND PROPHYLAKTISCHEN IMMUNSTIMULATION IN MENSCHEN UND HÖHEREN TIEREN**
SUBSTITUTED, NON-CODING NUCLEIC ACID MOLECULE USED FOR THE THERAPEUTIC AND PROPHYLACTIC IMMUNE STIMULATION IN HUMANS AND HIGHER ANIMALS
MOLECULE D'ACIDE NUCLEIQUE SUBSTITUEE ET NON CODANTE POUR LA STIMULATION IMMUNITAIRE THERAPEUTIQUE ET PROPHYLACTIQUE CHEZ L'HOMME ET LES ANIMAUX SUPERIEURS

(30) Priorität: 20.02.2004 WO PCT/DE2004/000361
(43) Veröffentlichungstag der Anmeldung: 02.11.2006
(73) Patentinhaber: Mologen AG, 14195 Berlin (DE)
(72) Erfinder: BRZEZICHA, Bernadette, 10555 Berlin (DE); JUHLS, Christiane, 10627 Berlin (DE); SACK, Florian, 12209 Berlin (DE); SCHMIDT, Manuel, 14195 Berlin (DE); WITTIG, Burghardt, 14129 Berlin (DE); SCHROFF, Matthias, 14057 Berlin (DE)
(74) Vertreter: Tegethoff, Sebastian
(86) Internationale Anmeldenummer: PCT/DE2004/001096
(87) Internationale Veröffentlichungsnummer: WO 2005/080567

(56) Entgegenhaltungen:
- WO-A-01/07055
- WO-A-02/060476
- WO-A-03/031469
- WO-A-03/031470
- SCHIRMBECK R ET AL: "Priming of immune responses to hepatitis B surface antigen with minimal DNA expression constructs modified with a nuclear localization signal peptide" JOURNAL OF MOLECULAR MEDICINE, Bd. 79, Nr. 5-6, Juni 2001 (2001-06), Seiten 343-350, XP002252260 ISSN: 0946-2716 in der Anmeldung erwähnt
- WITTIG B ET AL: "THERAPEUTIC VACCINATION AGAINST METASTATIC CARCINOMA BY EXPRESSION-MODULATED AND IMMUNOMODIFIED AUTOLOGOUS TUMOR CELLS: A FIRST CLINICAL PHASE I/II TRIAL" HUMAN GENE THERAPY, Bd. 12, Nr. 3, 10. Februar 2001 (2001-02-10), Seiten 267-278, XP001097188 ISSN: 1043-0342
- SCHMIDT MANUEL ET AL: "Immune-modulatory function of CpG sequence motifs in covalently-closed, double-stem-loop DNA constructs (dSLIM)." BLOOD, Bd. 102, Nr. 11, 16. November 2003 (2003-11-16), Seiten 769a-770a, XP001202623 ISSN: 0006-4971 & 45TH ANNUAL MEETING OF THE AMERICAN SOCIETY OF HEMATOLOGY; SAN DIEGO, CA, USA; DECEMBER 06-09, 2003
- RAGIN A D ET AL: "Cellular Import Mediated by Nuclear Localization Signal Peptide Sequences", CHEMISTRY AND BIOLOGY, vol. 9, no. 8, 1 August 2002 (2002-08-01), pages 943-948, XP027353364, ISSN: 1074-5521
- alberts, b. et al.: "Molecular biology of the cell, fifth edition", garland science page 707,

### SEQUENCE LISTING

<110> Mologen AG
<120> SUBSTITUIERTES, NICHT-KODIERENDES NUKLEINSÄUREMOLEKÜL ZUR THERAPEUTISCHEN
   UND PROPHYLAKTISCHEN IMMUNSTIMULATION IN MENSCHEN UND HÖHEREN TIEREN
<130> XI 302-04
<150> PCT/DE2004/000361
   <151> 2004-02-20
<160> 7
<170> PatentIn version 3.1
<210> 1
   <211> 58
   <212> DNA
   <213> Artificial
<220>
   <221> misc_feature
   <222> (9) .. (9)
   <223> synthetisches Oligodesoxynukleotid; T = chemisch modifiziertes
   Thymin
<400> 1
   tcccgtggtt accaccttca ttggaaaacg ttcttcgggg cgttcttagg tggtaacc 58
<210> 2
   <211> 58
   <212> DNA
   <213> Artificial
<220>
   <221> misc_feature
   <222> (9) .. (9)
   <223> synthetisches Oligodesoxynukleotid; T = chemisch modifiziertes
   Thymin
<400> 2
   acgggaggtt accaccttca ttggaaaacg ttcttcgggg cgttcttagg tggtaacc 58
<210> 3
   <211> 58
   <212> DNA
   <213> Artificial
<220>
   <221> misc_feature
   <223> unmodifiziertes synthetisches Oligodesoxynukleotid
<400> 3
   cctaggggtt accaccttca ttggaaaacg ttcttcgggg cgttcttagg tggtaacc 58
<210> 4
   <211> 56
   <212> DNA
   <213> Artificial
<220>
   <221> misc_feature
   <223> synthetisches Oligodesoxynukleotid
<400> 4
   tccctgttac caccttcatt ggaaaacgtt cttcggggcg ttcttaggtg gtaaca 56
<210> 5
   <211> 20
   <212> DNA
   <213> Artificial
<220>
   <221> misc_feature
   <222> (12)..(12)
   <223> synthetisches Oligodesoxynukleotid; T = chemisch modifiziertes
   Thymin
<400> 5
   gggagtccag ttttctggac 20
<210> 6
   <211> 12
   <212> PRT
   <213> Simian virus 40
<220>
   <221> MISC_FEATURE
   <223> NLS Peptid
<400> 6
   Pro Lys Lys Lys Arg Lys Val Glu Asp Pro Tyr Cys
   1               5                   10
<210> 7
   <211> 20
   <212> DNA
   <213> Artificial
<220>
   <221> misc_feature
   <223> synthetisches Oligodesoxynukleotid
<400> 7
   aggggtccag ttttctggac 20

## Beschreibung

Die Erfindung betrifft ein substituiertes, nicht-kodierendes Nukleinsäuremoleküle zur Modulation der Aktivität des menschlichen oder tierischen Immunsystems sowie ein Herstellungsverfahren desselben und eine Vakzine, welche das substituierte, nicht-kodierende Nukleinsäuremolekül enthält.

Bei der Ausprägung der Immunantwort gegen ein Antigen werden zwei Hauptarme des Immunsystems unterschieden: Zum einen, der humorale Arm welcher auf der Synthese von Antikörpern durch B-Lymphozyten aber auch humoralen Komponenten der nicht-adaptiven Immunität, dem Komplementsystem, beruht. Zum Anderen, der zelluläre Arm, welcher auf der Aktivität von T-Lymphozyten, NK-Zellen und antigenpräsentierenden Zellen des Immunsystems beruht. T-Lymphozyten sind in der Lage, mit Viren infizierte Zellen zu erkennen. Entsprechend wird der zelluläre Arm auch als T_{H}1 Antwort und der humorale Arm als T_{H}2 Antwort bezeichnet.

Extrazellulär vorliegende Bakterien und Parasiten werden in der Regel durch den T_{H}2 Arm bekämpft. Infektionserreger, die sich vor allem intrazellulär aufhalten, wie es für einzelne Bakterienarten und alle Viren bekannt ist, werden dagegen hauptsächlich durch den T_{H}1 Arm, also durch zytotoxische Zellen, bekämpft.

Die Impfstoffentwicklung hat in den letzten Jahren kontinuierlich wirksamere und zugleich nebenwirkungsärmere Vakzine hervorgebracht. Ausgehend von attenuierten Lebendimpfstoffen bzw. inaktivierten Pathogenen, wurden Impfstoffe entwickelt, die aus rekombinant hergestellten Antigenen bestehen (z.B. gegen Hepatitis B-Virus; rekombinante Impfstoffe gegen Herpes simplex Virus, Humanes Papillomvirus (Zervixkarzinom) und Borrelien sind in der klinischen Entwicklung). Diese Impfstoffe führen vor allem zu einer T_{H}2-dominierten Immunantwort und bedürfen der Ko-applikation von Adjuvantien, um eine stabile Immunität zu erreichen. Diese Adjuvantien führen dann zu einer Balance zwischen humoraler und zellulärer Immunantwort.

Einige Infektionskrankheiten können nur dann durch Impfung vermieden werden, wenn der Impfstoff eine T_{H}1-dominierte Immunantwort induziert. Für solche Impfstoffe wird derzeit die Kombination einer DNA-Vakzine (Induktion zellulärer Immunität) mit einem rekombinanten Antigen (Induktion humoraler Immunität) als beste Kombination gesehen (Esteban et al., Vaccine 2002, 20: 1226-1231). Dabei stellt das rekombinante Protein, das aufwendig und teuer in der Herstellung ist, den entscheidenden Kostenfaktor dar. Peptide, die preiswerter in der Herstellung sind, eignen sich nur bedingt zur Immunisierung, da sie eine Verschiebung zur T_{H}2-dominierten Immunantwort induzieren.

Bei allen Fortschritten in der DNA-Vakzinierung ist ein richtiger Durchbruch bis heute nicht erreicht worden. Dafür gibt es verschiedene Ursachen, so eine unzureichende Transfektion und ein damit verbundener niedriger Expressionslevel des verabreichten Antigens, sowie eine insgesamt unzureichend ausgeprägte Immunantwort als Ergebnis der Impfung.

Um die unzureichende Transfektionseffizienz zu verbessern, wurden verschiedene Peptide und andere organische Moleküle an Genfähren über unterschiedliche Bindungsarten gekoppelt. Ebenso wurde versucht, durch Kopplung von Liganden, die Ligand-Rezeptor Wechselwirkungen zur verstärkten Aufnahme der Genfähren auszunutzen (Fraser et al., 1998, Semin. Immunl., 10 (5): 363-72).

Durch kovalente Kopplung des Kernlokalisationssignals (NLS) aus dem SV 40 Virus an für das Hepatitis small surface Antigen (HBsAg) kodierende Expressionskassetten, konnte beispielsweise ein 10- bis 15-fach erhöhter Antikörpertiter nach intramuskulärer Applikation nachgewiesen werden (Schirmbeck et al., J. Mol Med. 2001 Jun;79 (5-6):343-50).

Um die Nachteile der heutigen viralen und plasmidbasierten Gentransfersysteme zu vermeiden, wurden kovalent geschlossene, minimalistische DNA-Expressionskonstrukte entwickelt (vgl. EP 0 914 318 B1). Diese minimalistischen Expressionskonstrukte können ebenfalls mit einem Kernlokalisationssignal aus dem SV40 Virus kovalent gekoppelt sein und führen dann zu einer Verschiebung in Richtung T_{H}1-betonte Immunantwort (vgl. WO 03/031469).

Zur Erzeugung einer verbesserten Immunantwort wurden CpG-Oligonukleotide (CpG-ODN) als neue Klasse von immunmodulatorischen Molekülen eingesetzt. Solche nicht methylierten CpG -Motive kommen in bakterieller DNA vor und stellen für das Immunsystem ein "danger signal" dar. Als "pathogen associated molecular pattern" (PAMP) bewirken sie vor allem die unspezifische Aktivierung des angeborenen Immunsystems (Krieg, Nat. Med 2003, 9: 831-835).

CpG-ODN induzieren über die Zytokine Interleukin-12, Interferon-gamma und Tumornekrosefaktor-alpha ebenfalls eine TH₁-betonte Immunantwort.

Immunstimulatorische Nukleinsäuresequenzen (ISS), die die beschriebenen CpG-ODN tragen, sind nur einige Basen lang und wirken nicht über die Expression von auf ihnen kodierten Proteinen.

Bei den eingesetzten ISS handelt es sich um hantelförmige, kovalent geschlosseneDesoxyribonukleinsäuremoleküle. Sie bestehen aus Oligonukleotiden, deren Basen partiell mit sich selbst paaren können und einer oder zwei Haarnadelschleifen (Loop), die 30 Basen umfassen und mehrere CpG -Motive enthalten (vgl. EP 1 196 178). Es wurde gezeigt, dass die immunmodulierende Wirkung dieser hantelförmigen, kovalent geschlossenen Desoxyribonukleinsäuremoleküle stark von der Loop-Doppelstrang-Kombination sowie den enthaltenen CpG Motiven abhängig ist. Die Linearisierung, die Deletion einer Haarnadelschleife, die Verkleinerung des Moleküls oder die Entfernung von CpG Motiven aus dem Molekül führte zu einem veränder- ten Induktionsmuster von Zytokinen.

Die starke Stimulation der zellulären Immunantwort ermöglicht eine Einflussnahme auf Regelkreisläufe, die ohne Eingriff nicht zu einer für den Patienten befriedigenden Immunaktivität führen.

Diese hantelförmigen, kovalent geschlossenen und nicht-kodierenden Nukleinsäuremoleküle, welche mindestens ein oder mehrere CpG Motive aufweisen und an einem oder beiden Enden einzelsträngige Haarnadelschleifen aufweisen, werden im weiteren synonym auch als Trägermoleküle bezeichnet.

Unter Nukleinsäuren sollen Desoxyribonukleinsäuren verstanden werden.

Immunstimmulation heißt im Zusammenhang dieser Erfindung, dass die Mittler- und Effektorzellen des Immunsystems, also vor allem die zur Zeit bekannten Thymozyten mit Helferfunktion und die zytotoxischen Thymozyten, B-Zellen und sog. NK (natural killer)-Zellen, Makrophagen und Monozyten sowie dendritische Zellen und ihre Vorläufer, sowie bisher in ihrer Funktion nicht aufgeklärte Zellpopulationen mit Funktionen innerhalb des Immunsystems, durch die Verwendung von Nukleinsäuremolekülen zur Proliferation, Migration, Differenzierung oder Aktivität angeregt werden. Immunmodulation heißt, dass neben einer generellen Stimulation im oben definierten Sinne auch die Art oder der Charakter einer Immunreaktion beeinflusst wird, sei es, dass eine im Entstehen oder der Reifung begriffene Immunreaktion davon betroffen ist, sei es, dass eine schon etablierte Reaktion in ihrem Charakter verändert wird.

Unter kurzen Nukleinsäuremolekülen werden im Sinne der Erfindung solche verstanden, die eine Kettenlänge gemäß den Ausführungsbeispielen (bevorzugt 48 bis 116 Nukleotiden) aufweisen.

Ausgehend von diesem Stand der Technik ist es die Aufgabe der vorliegenden Erfindung, geeignet modifizierte Trägermoleküle, die zur Auslösung einer wirkungsvollen Immunantwort fähig sind, sowie ein Verfahren zu ihrer Herstellung sowie Impfstoffe bereitzustellen, welche die modifizierten Trägermoleküle enthalten.

Die vorliegende Erfindung löst die Aufgabe dadurch, dass sie ein nicht-kodierendes Nukleinsäuremolekül gemäß Anspruch 1 zur Verfügung stellt, welches aus einer teilweise einzelsträngigen, hantelförmigen, kovalent geschlossenen Kette von Desoxyribonukleotidresten besteht und eine oder mehrere Sequenzen der Basenfolge N¹N²CGN³N⁴ im einzel- und / oder doppelsträngigen Bereich enthält, wobei N¹N² ein Element aus der Gruppe GT, GG, GA, AT oder AA, N³N⁴ ein Element aus der Gruppe CT oder TT, sowie C Desoxycytosin, G Desoxyguanosin, A Desoxyadenosin und T Desoxythymidin ist und welches mit einem oder mehreren Substituenten durch kovalente Bindungen mit dem nicht kodierenden Desoxyribonukleinsäuremolekül verknüpft ist, wobei wenigstens ein Substituent ein Kernlokalisationssignal gemäß Seq. ID 6 ist.

Je nach Ausführung des Desoxyribonukleinsäuremoleküls ist der zum Kernlokalisationssignal unterschiedliche kovalent gebun-dene Substituent aus der Gruppe der Peptide (einschließlich kationischer Peptide), Proteine, Saccharide, antigenen Strukturen, DNA und / oder RNA ausgewählt.

In einer Ausführungsform des erfindungsgemäßen Nukleinsäuremoleküls bei der mehr als ein Substituent kovalent gebunden ist, können diese entweder aus der gleichen oder aus verschiedenen Gruppen ausgewählt sein. Für den Fall, dass ein solches Desoxyribonukleinsäuremolekül mit mehreren Substituenten kovalent verbunden ist, können diese identisch oder voneinander verschieden sein.

Bei einem erfindungsgemäßen Nukleinsäuremolekül sind sowohl der oder die Substituenten chemisch modifiziert als auch die Nukleotide, mit denen die Substituenten verbunden sind. Es ist aber auch denkbar, dass nur einer der beiden Partner chemisch modifiziert werden muss, um die kovalente Bindung herbeizuführen. Des weiteren ist es auch möglich, dass die Bindung zwischen Substituent und Nukleotid über ein bifunktionales Kopplungsreagenz zustande kommt.

Die erfindungsgemäßen Nukleinsäuremoleküle werden durch ein Verfahren gemäß Anspruch 8 hergestellt. Dieses Herstellungsverfahren ist durch folgende Verfahrensschritte gekennzeichnet:
a. Zwei Oligodesoxyribonukleotide, welche partiell selbstkomplementär sind und von denen wenigstens eines chemisch modifiziert ist, werden unter geeigneten Reaktionsbedingungen zumindest mit dem Kernlokalisationssignal gemäß Seq. ID 6 gekoppelt,
b. anschließend wird den aufgereinigten Produkten der Kopplungsreaktion durch kurzes Erhitzen und Abkühlen auf Eis die partielle Basenpaarung sowie das Ausbilden von Haarnadelstrukturen ermöglicht, und
c. die daraus resultierenden selbstkomplementären Überhänge der teilweise doppelsträngigen nicht-kodierenden Nukleinsäuremoleküle werden miteinander oder mit Oligodesoxynukleotiden, welche mit anderen Substituenten kovalent verbunden sind, ligiert,
d. gefolgt von einer Exonukleasebehandlung zum Abbau von nicht kovalent geschlossenen Ketten aus substituentgekoppelten Oligodesoxyribonukleotide, bevor
e. abschließend die nicht-kodierenden, substituentgekoppelten Nukleinsäuremoleküle aufgereinigt werden.

Bei dem erfindungsgemäßen Verfahren können Oligodesoxynukleotide zur Kopplung eines Substituenten verwendet werden, die durch eine oder mehrere alkylische Carbonsäure-, Amin-, Thiol- oder Aldehydfunktionen oder eine Maleimidsäuregruppe chemisch modifiziert sind. Je nach Ausführungsform des herzustellenden Nukleinsäuremoleküls können auch Substituenten verwendet werden, die durch eine oder mehrere alkylische Carbonsäure-, Amin-, Thiol- oder Aldehydfunktionen oder eine Maleimidsäuregruppe chemisch modifiziert sind.

Erfindungsgemäße Nukleinsäuremoleküle können als Adjuvans in der therapeutischen oder prophylaktischen Vakzinierung in Menschen oder höheren Tieren ver-wendet werden. Des weiteren ist eine Verwendung der erfindungsgemäßen Nukleinsäuremoleküle für die Immunstimulation in Menschen oder höheren Tieren denkbar, wobei diese *in vitro* oder *in vivo* erfolgen kann.

Die erfindungsgemäßen Nukleinsäuremoleküle können auch Bestandteil eines Impfstoffes sein, der zur Modulation der Immunantwort in Menschen oder höheren Tieren eingesetzt wird.

Dieser Impfstoff, der aus einer Kombination von erfindungsgemäßen Nukleinsäuremolekülen mit substituentgekoppelten, hantelförmigen, linear kovalent geschlossenen DNA-Expressionskonstrukten besteht, ist ebenfalls vorgesehen. Diese Kombinationsvakzine kann als Impfstoff in der therapeutischen oder prophylaktischen Vakzinierung in Menschen oder höheren Tieren oder als Adjuvans für die Immunstimulation in Menschen oder höheren Tieren, welche *in vitro* oder *in vivo* erfolgen kann, eingesetzt werden.

Erfindungsgemäß wird demnach die oben geschilderte Aufgabe dadurch gelöst, dass kurze nicht-kodierende Nukleinsäuremoleküle hergestellt werden, die mindestens eine oder mehrere CpG Sequenzen tragen, auch als Trägermoleküle bezeichnet, und durch kovalente Kopplung mit mindestens einem oder mehreren Substituenten aus der Klasse der Peptide (einschließlich kationischer Peptide), Proteine, Saccharide, antigenen Strukturen, DNA und / oder RNA verbunden sind, wobei wenigstens ein Substituent ein Kernlokalisationssignal gemäß Seq. ID 6 ist. Des weiteren ist die Bereitstellung einer neuen Vakzinekombination bestehend aus erfindungsgemäßen substituentgekoppelten Trägermolekülen und minimalistischen substituentmodifizlerten DNA-Expressionsvektoren vorgesehen.

Diese kurzen nicht-kodierenden Nukleinsäuremoleküle, bestehen aus einer teilweise einsträngigen, hantelförmlgen, kovalent geschlossenen Folge von Nukleosidresten und tragen eine oder mehr Sequenzen der Basenfolge N¹N²CGN³N⁴, wobei N¹N² ein Element aus der Gruppe GT, GG, GA, AT oder AA, N³N⁴ ein Element aus der Gruppe CT oder TT, sowie C Desoxycytosin, G Desoxyguanosin, A Desoxyadenosin und T Desoxythymidin ist. Solche kovalent geschlossenen nicht-kodierenden Nukleinsäuremoleküle kann man aus offenkettigen Nukleinsäuremolekülen, welche eine in Teilen selbstkomplementäre Sequenz aufweisen und miteinander oder einem zweiten Molekül ein intermediär stabiles Hybrid bilden können, welches einen geschlossenen Doppelstrangbereich mit einer Lücke im Zucker-Phosphatrückgrat aufweist, durch Ligation dieser Lücke im Rückgrat durch ein geeignetes Enzym, etwa die DNA-Ligase aus dem Bakteriophagen T4, gewinnen. Alternativ kann das Trägermolekül auch durch intramolekulare Ligation eines Moleküls, weiches mindestens zwei selbstkomplementäre Bereiche aufweist, welche durch nur eine Lücke im Phosphatrückgrat getrennt sind, gewonnen werden.

Wesentlich ist mithin die kovalente Kopplung von Substituenten an dieses Trägermolekül und die sich daraus ergebenden neuen Eigenschaften des entsprechenden Moleküls. Dabei kann die Kopplung an beliebige Nukleotide des Stammes oder der Loops (der Haarnadelschleifen) erfolgen. Im unten beschriebenen Ausführungsbeispiel erfolgt die kovalente Kopplung über eine eingefügte Sulfhydrylgruppe (SH-Gruppe) am Thymin, ebenso ist die Kopplung über ein bifunktionelles Kopplungsre-agenz denkbar. Andere Varianten sind möglich und durch die Beschreibung nicht ausgeschlossen. Die unten gezeigten Beispiele sind daher nicht abschließend.

Zur zusätzlichen Kopplung eignet sich jeder Substituent aus der Gruppe der Peptide (einschließlich kationischer Peptide), Proteine, Saccharide, antigenen Strukturen, DNA und / oder RNA, der zuvor mit einer Maleimidgruppe am aminoterminalen Ende versehen wurde.

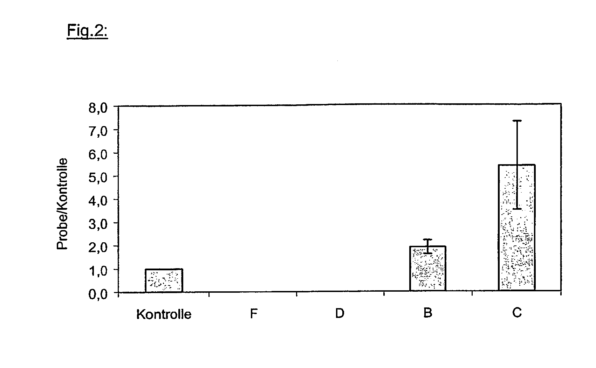

Unter Substituent soll beispielhaft das NLS-Peptid (nuclear-localization sequence aus dem SV-40 Virus) verstanden werden. Die kovalente Verbindung von Trägermolekül mit dem NLS-Peptid führt durch die beschriebenen Strukturmerkmale (Doppelstrang-Haarnadelschleifen-Kombination, CpG -ODN) zu einem synergistischen Effekt bei der Induktion einer Immunantwort. Der adjuvantive Effekt der Trägermoleküle wird durch die Kopplung des NLS-Peptides maßgeblich verstärkt. Bei in-vitro Stimulationsversuchen mit B-Zellen, konnte bei Verwendung der erfindungsgemäßen Moleküle im Vergleich zur Kontrollgruppe eine über 50%ige Steigerung der Expression des Oberflächenmarkers CD80 erreicht werden (siehe Fig. 2).

Unter Substituent sollen weiterhin Antigene oder deren Epitope, die zur Auslösung einer schützenden Immunität geeignet sind, verstanden werden. Darunter fallen beispielshaft und nicht abschließend Antigenepitope des humanen Papilloma Virus, des Influenza Virus, beispielsweise immunogene Epitope der Proteine H, M1 und NP, des SARS Virus, hier beispielsweise immunogene Epitope der Proteine S, M, und N, des Tuberkulose Virus, des HI Virus, des Hepatitis-B-Virus, weiterhin Antigenepitope parasitärer Erkrankungen, wie bspw. der Leishmaniose sowie die Gruppe der tumorassoziierten Antigene (TAA), wie sie von tumoralen Erkrankungen bekannt sind.

Die Vorteile der Kopplung von Trägermolekülen mit einem oder mehreren Antigenepitopen, wahlweise im Doppelstrang oder im Haamadelbereich, liegen einerseits in der Induktion einer antigen-spezifischen Immunantwort des erworbenen Immunsystems und andererseits in einer Aktivierung des unspezifischen Immunsystems. Zusätzlich wird durch eine entsprechende Zytokinausschüttung eine T_{H}1-dominierte Immunantwort verstärkt. Diese kombinierte Induktion einer schnellen, unspezifischen Immunmodulation, ausgelöst vom Trägermolekül, mit einer verzögerten spezifischen Immunantwort, ausgelöst vom Substituenten, in diesem Fall von bestimmten Antigenepitopen, erlaubt es, diese Art von Molekülen auch bei akut bevorstehenden Infektionen einzusetzen.

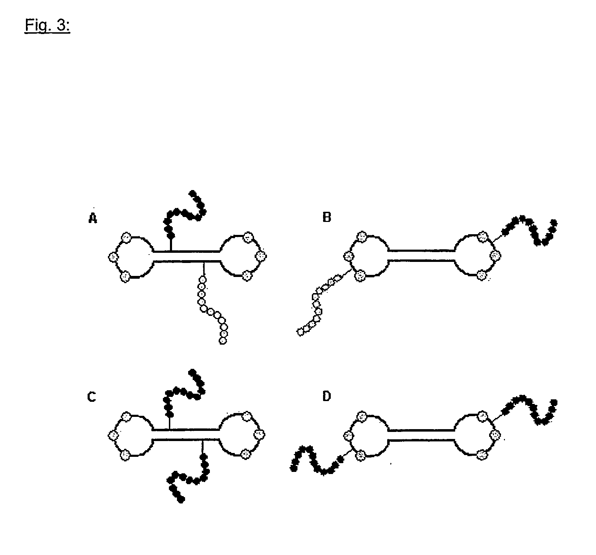

Die Kopplung von verschiedenen, nicht identischen, Substituenten an das Trägermolekül ist ausdrücklich vorgesehen. Beispiele dieser erfindungsgemäßen substituentgekoppelten Trägermoleküle sind in Fig. 3 - nicht abschließend - dargestellt.

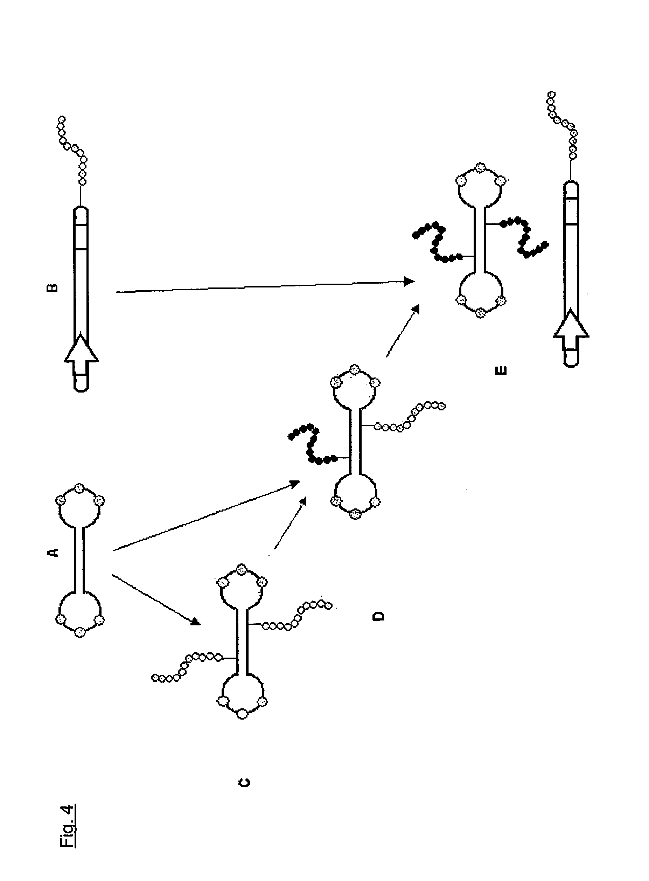

Ein weiterer Aspekt der Erfindung ist der kombinierte Einsatz der substituentgekoppelten Trägermoleküle mit minimalistischen, linearen, substituentgekoppelten DNA-Expressionsvektoren, die genetische Information für ein entsprechendes Antigen tragen. Diese erfindungsgemäße Kombinationsvakzine ist in Fig. 4 unter E dargestellt. An diese DNA-Expressionsvektoren können ein oder mehrere identische oder voneinander verschiedene Substituenten kovalent gekoppelt sein. Die Substituenten sind ausgewählt aus der Gruppe der Peptide (einschließlich kationischer Peptide), Proteine, Saccharide, antigenen Strukturen, DNA und / oder RNA. So kann es sich bei dem exprimierten Antigen und als Substituent gekoppelten Antigen um ein und dasselbe Antigen handeln. Es ist aber auch vorgesehen, das sich exprimiertes Antigen und am DNA-Expressionskonstrukt gekoppelter Substituent voneinander verschieden sind und aus verschiedenen Gruppen ausgewählt sind.

Ein derartiger Impfstoff kann durch die Komponente der erfindungsgemäßen substituentgekoppelten Trägermoleküle das angeborene Immunsystem aktivieren und ist so in der Lage, binnen 24 - 48 h Schutz vor viralen, parasitären und bakteriellen Infektionen zu gewähren. Die zweite Komponente, die substituentgekoppelten DNA-Expressionskonstrukte vermitteln antigen-spezifische Immunität. So kommt es zur spezifischen Antikörperproduktion und zur Ausbildung der zytotoxische T-Zell-Antwort innerhalb von 2 Wochen nach der Impfung.

Diese Vakzinekombination führt zu einem variablen Impfstoff aus antigenexprimierenden minimalistischen DNA-Expressionskonstrukten und substituentgekoppelten Trägermolekülen mit schnellem Wirkeintritt. Es kann sich bei dem am Trägermolekül und an dem DNA-Expressionskonstrukt gekoppelten Substituenten um den gleichen Substituenten handeln, der als Antigen vom DNA-Expressionsvektor exprimiert wird, es können jedoch auch von den Substituenten unterschiedliche Antigene sein, die exprimiert werden.

An die Trägermoleküle können ein oder mehrere identische oder verschiedene Substituenten zusätztlich zum Kernlokalisatiossignal gemäß Seq. Id 6 kovalent gekoppelt sein. Befinden sich identische Substituenten an einem Trägermolekül, so ist es auch vorgesehen, das diese ersten mit anderen Trägermolekülen, die davon verschiedene Substituenten aufweisen, gemischt werden, und diese Mischung dann bestehend aus verschiedenen Substituenten appliziert wird.

Die schematische Darstellung der erfindungsgemäßen Moleküle und der erfindungsgemäßen Kombination sind in Fig. 4 gezeigt.

Vorteilhafterweise weisen die erfindungsgemäßen Moleküle keine freien 5'- oder 3'-Enden auf und sind somit nicht für Exonukleasen zugänglich.

Die erfindungsgemäßen Nukleinsäuremoleküle können je nach Ausführung die Sequenz (SeqID.1) oder (SeqID.2) aufweisen.

Die beanspruchten Nukleinsäuremoleküle mit den oben genannten konkreten Sequenzen oder Sequenzbereichen weisen bevorzugt eine Länge von bis zu 200, besonders bevorzugt von 48 bis 116 Nukleotiden auf.

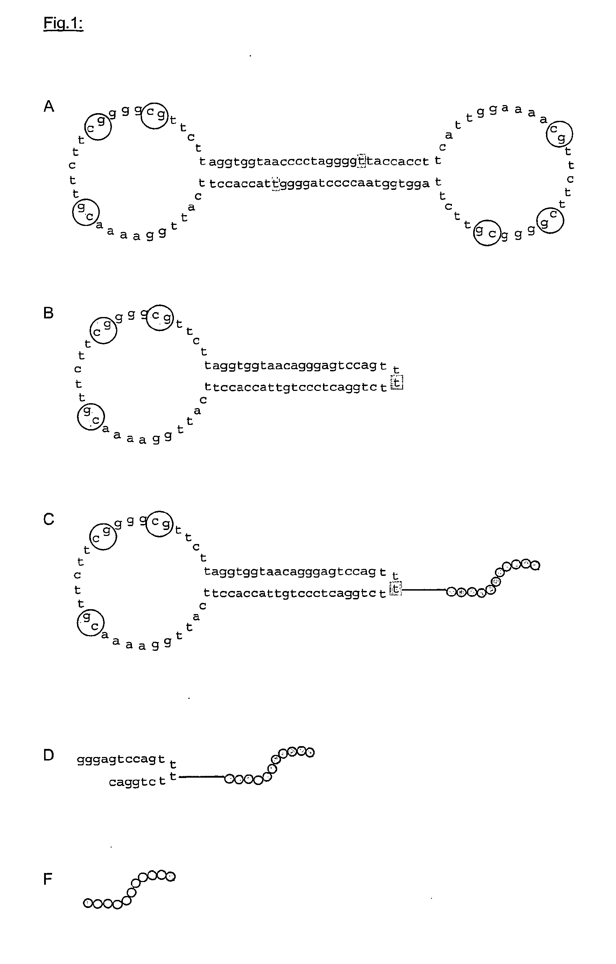

Weitere vorteilhafte Maßnahmen sind in den übrigen Unteransprüchen beschrieben; die Erfindung wird anhand der nachfolgenden Figuren und von Ausführungsbeispielen näher beschrieben
- **Fig.1**: zeigt beispielhaft mögliche Trägermoleküle sowie ein erfindungsgemäßes mit Substituenten gekoppeltes Trägermolekül. Ein Strukturmerkmal der Trägermoleküle, die CpG -Motive, sind in den Haamadelschleifen durch Kreise verdeutlicht. Das im Ausführungsbeispiel zur Kopplung der jeweiligen Substituenten verwendete Nukleotid Thymin ist grau hinterlegt. Wobei jedes andere Nukleotid gleich geeignet ist. Die Kopplung mit einem Substituenten kann an mehreren Stellen in den Haarnadelschleifen erfolgen, ebenso kann die Kopplung im Doppelstrangbereich der Trägermoleküle erfolgen. Im Einzelnen zeigt
A: Trägermolekül mit gekennzeichneten möglichen Kopplungspositionen im Doppelstrangbereich sowie drei CpG-Motiven in jeder Haarnadelschleife
B: Trägermolekül mit einer Haarnadelschleife
C: substituentgekoppeltes Trägermolekül mit einer Haarnadelschleife
D: zeigt einen an ODN gekoppelten Substituenten, der als Kontrolle im Ausführungsbeispiel 6 benutzt wurde
F: zeigt einen Substituenten, der als Kontrolle im Ausführungsbeispiel 6 benutzt wurde
- **Fig. 2**: Eine Überprüfung der immunstimulatorischen Effekte der substituentgekoppelten Trägermoleküle erfolgte im Rahmen eines Stimulationsversuches mit B-Zellen. Folgende Proben wurden eingesetzt: Kontrolle: B-Zellen ohne Zugabe von Proben
F: NLS-Peptid
D: ODN mit NLS-Peptid gekoppelt
B: Trägermolekül mit einer Haarnadelschleife
C: NLS-gekoppeltes Trägermolekül
Auf der Abszisse ist das Verhältnis zwischen Probenwert und Kontrollwert dargestellt. Die Balken im Diagramm zeigen die Ergebnisse der in-vitro Versuche anhand der stimulierten Expression des Oberflächenmarkers CD80. Die Proben F und D sind ebenfalls als Kontrollen eingesetzt und zeigten keine Stimulationswirkung. Das NLS-Peptid allein und das NLS-Peptid gebunden an ein ODN sind nicht in der Lage, B-Zellen zur Ausschüttung von CD80 anzuregen. Dagegen führt das Trägermolekül mit einer Haarnadelschleife und ohne NLS-Peptid zu einer verstärkten Ausschüttung von CD80. Mit dem erfindungsgemäßen NLS-gekoppelten Trägermolekül wird dieser Stimulationseffekt jedoch sogar um den Faktor 3 erhöht. Der Versuch zeigt, das erst die erfindungsgemäße Verbindung der Komponenten Doppelstrang-, einsträngiger Haarnadelschleifenbereich, CpG-Motive und die kovalente Kopplung von Substituenten, in diesem Fall NLS-Peptid, zu einem überraschenden Ergebnis führen.
- **Fig. 3**: zeigt schematisch Kopplungsmöglichkeiten gleicher und verschiedener Substituenten an Trägermoleküle; im Einzelnen zeigt
A: ein Trägermolekül mit zwei Haarnadelschleifen, darin enthaltenden CpG-Motiven, die schematisch als graue Kreise dargestellt sind und mit zwei voneinander verschiedenen Substituenten, die im Doppelstrangbereich des Trägermoleküls gebunden sind.
B: ein Trägermolekül mit zwei Haarnadelschleifen, darin enthaltenden CpGMotiven, die schematisch als graue Kreise dargestellt sind und mit zwei voneinander verschiedenen Substituenten, die im einsträngigen Haarnadelschleifenbereich des Trägermoleküls gebunden sind.
C: ein Trägermolekül mit zwei Haarnadelschleifen, darin enthaltenden CpGMotiven, die schematisch als graue Kreise dargestellt sind und mit zwei gleichen Substituenten, die im Doppelstrangbereich des Trägermoleküls gebunden sind.
D: ein Trägermolekül mit zwei Haarnadelschleifen, darin enthaltenden CpG-Motiven, die schematisch als graue Kreise dargestellt sind und mit zwei gleichen Substituenten, die im einsträngigen Haarnadelschleifenbereich des Trägermoleküls gebunden sind.
- **Fig. 4**: zeigt die erfindungsgemäßen substituentgekoppelten Trägermoleküle und die Kombinationsvakzine in einer Übersicht; im Einzelnen zeigt
A: ein Trägermolekül bestehend aus einsträngiger Haarnadelschleifen-Doppelstrang-Kombination, CpG-Motive enthaltend (schematisch dargestellt als Kreise).
B: ein minimalistisches, kovalent geschlossenes DNA-Expressionskonstrukt, das mit einem Substituenten gekoppelt ist.
C: ein substituentgekoppeltes Trägermolekül, das mit zwei gleichen Substituenten im Doppelstrangbereich gekoppelt ist.
D: ein substituentgekoppeltes Trägermolekül, das mit unterschiedlichen Substituenten im Doppelstrangbereich gekoppelt ist.
E: zeigt die erfindungsgemäße Kombination von substituentgekoppeltem Trägermolekül und substituentgekoppeltem DNA-Expressionskonstrukt. Die Substituenten beider Moleküle sind unterschiedlich, können aber auch gleich sein.

### Beispiel 1: Synthese der unmodifizierten Trägermoleküle entsprechend EP1196178

Phosphorylierte ODN (SeqID.3) der Sequenz CCTAGGGGTT ACCACCTTCA TTGGAAAACG TTCTTCGGGG CGTTCTTAGG TGGTAACC (TIB-Molbiol, Berlin) wurden 5 min auf 90°C erhitzt und anschließend auf Eis gekühlt, um die Ausbildung der Haarnadelstruktur zu ermöglichen. Selbstkomplementäre Überhänge wurden bei einer Endkonzentration von 1 Mikrogramm/Mikroliter DNA in Gegenwart von T4-DNA Ligase (0,1 U/Mikrogramm ODN) 24 h bei 37°C ligiert. Nach Phenolextraktion und anschließender Extraktion mit Chloroform sowie Präzipitation mit Isopropanol in Anwesenheit von MgCl₂ (f.c. 10 mM) und NaAc (f.c. 300 mM) wurde das Produkt nach Zentrifugation und Aufnehmen in Wasser erhalten.

Zur Reinigung von Endotoxinen wurde das Ligationsprodukt einer anschließenden Anionenaustauschchromatographie (Trägermaterial: LiChrospher DMAE, Merck Darmstadt; 0-1 M NaCl in 50 mM NaPhosphat) unterworfen und durch Isopropanolpräzipitation konzentriert. Für in-vivo-Versuche wird das Verfahren unter sterilen Bedingungen durchgeführt und das Endprodukt in sterilem PBS aufgenommen.

### Beispiel 2: Herstellung substituentgekoppelter Trägermoleküle mittels zweier verschiedener Herstellungswege a) und b)

### a) Kopplung von maleimid-funktionalisierte Substituenten an Thiol (SH) modifizierte Oligodesoxyribonukleotiden (SH-ODN)

Die Thiol-Gruppe des SH-ODN wurde wegen ihrer hohen Reaktivität mit der Schutzgruppe Dimethoxytrityl (DMT) versehen. Diese Schutzgruppe wird vor der Kopplungsreaktion entfernt. Für die Herstellung substituentgekoppelter Trägermoleküle werden von Bsp.1 verschiedene ODN benutzt, die eine definierte SH-ODN Modifizierung gewährleisten. Definierte Kopplung erfolgt entweder am Stamm oder in den Loops oder in Stamm und Loops, hierfür werden ODN 1 und ODN 2 benutzt:
ODN 1 (SeqID.1): und
ODN 2 (SeqID.2): (bezogen von Eurogentec, Belgien), wobei XT für SH-modifziertes T und Ph für 5'-Phosphorylierung steht.

Um die Schutzgruppe DMT zu entfernen, wurde das SH-ODN in Deprotektionspuffer (4mM K₂HPO4, 4mM KH₂PO₄, pH 6,8) gelöst und 120mM Silbernitrat (AgNO₃) zugegeben. Dieser Ansatz wurde 30min bei Raumtemperatur (RT) inkubiert. Anschlie-ßend wurde der Deprotektionsansatz mit 0,2 Volumenteilen DTT versetzt und 20min bei RT inkubiert. Der Reaktionsansatz wurde kurz zentrifugiert und der Überstand über eine Gelfiltrationssäule (NAP-5, Amersham Biosciences) gegeben zur Reinigung der nun entschützten SH-ODN.

Die entschützten SH-ODN wurden in Kopplungspuffer (100mM Na₂HPO₄, 150mM NaCl) mit dem maleimid-modifizierten Substituent in Reaktion gebracht. Der Ansatz wurde eine Stunde bei Raumtemperatur inkubiert, um die Maleimid-Gruppe mit der SH-Gruppe reagieren zu lassen. Prinzipiell ist die Verwendung anderer SH-Gruppen enthaltender ODN, geschützt sowie ungeschützt, möglich. Die Kopplung der SH-Gruppen mit dem Maleimid-Substituent stellt nur ein Beispiel für die Gruppe aller Maleimid enthaltender Substituenten dar.

### b) Kopplung von Thiol-Gruppen enthaltenden Substituenten über bifunktionelle Crosslinker an Amino-ODN

Zur konzeptionellen Überprüfung wurde hier die Reaktion einer Thiolgruppe, die am zu koppelnden Substituent vorhanden war, mit einer Maleinsäureimidgruppe am ODN verwendet. Die Maleinimidgruppe wurde über die Reaktion eines bei der Synthese des ODN eingefügten Aminorests (mit X gekennzeichnet) mit einem kommerziell erhältlichen bifunktionellen Kopplungsreagenz über eine Transamidierung des im Kopplungsreagenz vorliegenden NHS-Carbonsäurederivats in die Nukleinsäurekomponente der Reaktion eingeführt. Zur Herstellung wurden die aminomodifizierten ODN wie folgt verwendet:
ODN 1 (SeqID.1): und
ODN 2 (SeqID.2): (bezogen von Eurogentec, Belgien), wobei XT für NH-modifziertes T und Ph für 5'-Phosphorylierung steht.

Die aminomodifizierten ODN wurden wie folgt zur Kopplung eingesetzt: Der Crosslinker zur kovalenten Bindung (hier: sulfo-KMUS (N-(Maleimidoundecanoyloxy) sulfosuccinimid) in DMSO, PIERCE Produkt-Nr. 21111) wurde in vier gleichen Teilen im Abstand von 30min zu den Amino-ODN (0,1mM Endkonzentration) gegeben, bis eine Endkonzentration von 5 mM erreicht war. Die Reaktion erfolgte für zwei Stunden in einem Crosslink-Kopplungspuffer (50mM NaHPO₄, 75mM NaCl, pH 7,6) bei 37°C. Anschließend wurde die Reaktion durch Zugabe von Tris-HCl (pH 7,5; 50 mM Endkonzentration) gestoppt. Die aktivierten Amino-ODN wurden für 30min bei -70 C Ethanol-präzipitiert (300 mM NaOAc pH 5,3; 20 mM MgCl₂; 2,5-faches Reaktionsvolumen 100 % Ethanol abs.). Das Präzipitat wurde 30min bei 15.000 rpm (4 C) zentrifugiert und unter gleichen Bedingungen für 15min mit 70 % Ethanol gewaschen. Die aktivierten Amino-ODN wurden in Kopplungspuffer (1x = 50 mM NaHPO₄, 75 mM NaCl, pH 7,0) aufgenommen, so dass sich eine 0,1 millimolare Endkonzentration ergab. Anschließend wurde der in Wasser gelöste Thiol-Substituent (bezogen von Peter Henklein, Charité, Berlin) in 0,2 mM Endkonzentration zugegeben. Die Reaktion erfolgte bei 37°C für eine Stunde.

Die Reinigung und Trennung der resultierenden substituentgekoppelten ODN von den nicht gekoppelten ODN erfolgte mittels Reversed Phase-HPLC. Die einzelnen Reaktionen wurden gelelektrophoretisch analysiert. Die HPLC-Fraktion mit den substituentgekoppelten ODN wurde in einer Vakuumzentrifuge eingeengt und in MilliQ-Wasser aufgenommen. Die substituentgekoppelten ODN wurden über eine Nukleosil-300 C18 Säule (10Mikromol, 250mm Länge x 8mm Innendurchmesser) HPLC gereinigt. Der Gradient verlief von 0% Puffer A (100mM Ammoniumcarbonat) auf 42% Puffer B (80% Acetonitril) in 47min bei einer Flussrate von 2,4ml/min.

Die entsprechend Beispiel 2a und b hergestellten substituentgekoppelten ODN wurden 5 min auf 90°C erhitzt und anschließend auf Eis gekühlt, um die Ausbildung der Haarnadelstruktur zu ermöglichen. Selbstkomplementäre Überhänge wurden bei einer Endkonzentration von 0,55 Mikrogramm/Mikroliter DNA in Gegenwart von T4-DNA Ligase (0,01 U/Mikrogramm ODN) 24 h bei 37°C ligiert. Nach Hitzeinaktivierung (68 - 72°C) der T4-DNA Ligase für 20min folgte ein T7-DNA-Polymeraseverdau (0,025 U/Mikrogramm ODN) zur Entfernung nicht kovalent geschlossener substituentgekoppelten ODN für mindestens 12h bei 37°C. Nach Phenolextraktion und anschließender Extraktion mit Chloroform sowie Präzipitation mit Isopropanol in Anwesenheit von MgCl₂ (f.c. 10 mM) und NaAc (f.c. 300 mM) wurde das Produkt nach Zentrifugation und Aufnehmen in Wasser erhalten. Zur Reinigung des Produkts wurde das Ligationsprodukt einer anschließenden Anionenaustauschchromatographie (Trägermaterial: Fractogel DMAE(s), Merck Darmstadt; 0-1 M NaCl in 20 mM Tris/HCl pH7,0)) unterworfen und durch Isopropanolpräzipitation konzentriert. Für in-vivo-Versuche wird das Verfahren unter sterilen Bedingungen durchgeführt und das Endprodukt in sterilem PBS aufgenommen.

Beispiel 3: Herstellung von substituentgekoppelten Trägermolekülen mit einem Loop
ODN 3 (SeqID.4): und
ODN 4 (SeqID.5):
   5'Ph-GGG AGT CCA GT XTT TCTG GAC
(bezogen von Eurogentec, Belgien), wobei XT für NH-modifziertes T und Ph für 5'-Phosphorylierung steht.

Das ODN 4 (SeqID.5) wurde nach dem in 2b beschriebenen Kopplungsmechanismus über die NH-Gruppe und den bifunktionellen Crosslinker sulfo-KMUS an das Peptid NLS (PKKKRKVEDPYC (SeqID.6, bezogen von Peter Henklein, Charité, Berlin) über das Thiol der Cysteingruppe gekoppelt. Die Ligation und die weiteren Schritte erfolgten wie beschrieben.

### Beispiel 4: Herstellung minimalistischer, kovalent geschlossener DNA-Expressionskonstrukte für die Kombinationsvakzine

Die verwendeten DNA-Expressionskonstrukte sind lineare, kovalent geschlossene Expressionskassetten, die aus dem CMV-Promotor, einem Intron, der für ein Antigen kodierenden Gensequenz und einer Polyadenylierungssequenz bestehen (vgl. EP 0 941 318 B1). Ein geeignetes Plasmid, das die betreffende Gensequenz trägt, wurde mit Eco 31I vollständig verdaut. Die Ligation mit 5' phosphorylierten haarnadelförmigen
ODN 5 (SeqID.7):
   5'-Ph-AGG GGT CCA GTT TTC TGG AC (bezogen von Eurogentec, Belgien)
erfolgte durch T4 DNA Ligase in Anwesenheit von Eco 31l und wurde durch Erhitzen auf 70°C gestoppt. Das resultierende Gemisch wurde konzentriert und mit Eco 31l und T7 DNA Ligase in Abwesenheit von Deoxyribonukleotid-Triphosphaten behandelt. Die Aufreinigung erfolgte durch Anionenaustauschchromatographie.

### Beispiel 5: Herstellung minimalistischer, kovalent geschlossener DNA-Expressionskonstrukte mit Substituent

Zur Herstellung minimalistischer substituentgekoppelter DNA-Expressionskonstrukte, in diesem Beispiel mit NLS-Peptid als Substituent, wurden das in der Haarnadelschlaufe mit einem aminogruppenmodifizierten desoxy-Uracil (XT) versehene ODN 4 (SeqID.5) sowie das nichtmodifizierte ODN 5 (SeqID.7) verwendet:
ODN 4 (SeqID.5):
   5'-Ph-GGG AGT CCA GT XT TTC TGG AC
   (bezogen von Eurogentec, Belgien), wobei XT für NH-modifziertes T und Ph für 5'-Phosphorylierung steht.
   und
ODN 5 (SeqID.7):
   5'-Ph-AGG GGT CCA GTT TTC TGG AC (bezogen von Eurogentec, Belgien)

Das aminomodifizierte ODN 4 wurde wie folgt zur Kopplung eingesetzt: Der Crosslinker zur kovalenten Bindung (hier: sulfo-KMUS (N-(Maleimidoundecanoyloxy) sulfosuccinimid) in DMF, PIERCE Produkt-Nr. 21111) wurde in vier gleichen Teilen im Abstand von 30 min zu den Amino-ODN (0,1mM Endkonzentration) gegeben, bis eine Endkonzentration von 5 mM erreicht war. Die Reaktion erfolgte für zwei Stunden in einem Crosslink-Kopplungspuffer (50mM NaHPO₄, 75mM NaCl, pH 7,6) bei 37°C. Anschließend wurde die Reaktion durch Zugabe von Tris-HCl (pH 7,5; 50 mM Endkonzentration) gestoppt. Die aktivierten Amino-ODN wurden für 30 min bei -70 C Ethanol-präzipitiert (300 mM NaOAc pH 5,3; 20 mM MgCl₂; 2,5-faches Reaktionsvolumen 100 % Ethanol abs.). Das Präzipitat wurde 30 min bei 15.000 rpm (4 C) zentrifugiert und unter gleichen Bedingungen für 15 min mit 70 % Ethanol gewaschen. Die aktivierten Amino-ODN wurden abschließend in Wasser (MilliQ-Qualität) aufgenommen und bis zur Kopplung bei -20 C eingefroren.

### Beispiel 5a :Kopplung von NLS- Peptid an aktivierte ODN

Die unter 5 beschriebenen aktivierten Amino-ODN wurden in Kopptungspuffer (1x = 50 mM NaHPO₄, 75 mM NaCl, pH 7,0) aufgenommen, so dass sich eine 0,1 millimolare Endkonzentration ergab. Anschließend wurde das in Wasser gelöste NLS Peptid PKKKRKVEDPYC (bezogen von Peter Henklein, Charité, Berlin) in 0,2 mM Endkonzentration zugegeben. Die Reaktion erfolgte bei 37°C für eine Stunde. Die Reinigung und Trennung der resultierenden NLS-gekoppelten ODN von den nicht gekoppelten ODN erfolgte mittels Reversed Phase-HPLC. Die einzelnen Reaktionen wurden gelelektrophoretisch analysiert. Die HPLC-Fraktion mit den NLS-ODN wurde in einer Vakuumzentrifuge eingeengt und in MilliQ-Wasser aufgenommen. Die modifizierten ODN wurden über eine Nukleosil-300 C18 Säule (10Mikromol, 250mm Länge x 8mm Innendurchmesser) HPLC gereinigt. Der Gradient verlief von 0% Puffer A (100mM Ammoniumcarbonat) auf 42% Puffer B (80% Acetonitril) in 47min bei einer Flussrate von 2,4ml/min.

Die minimalistischen, kovalent geschlossenen DNA-Expressionskonstrukte mit NLS-Substituent wurden wie folgt erhalten: nach Erhalt der Expressionskassette durch Eco 31I Verdau, entsprechend Beispiel 4, erfolgte die Ligation mit den vorbereiteten ODN. Die Ligation mit 5' phosphorylierten haarnadelförmigen ODN 4, an das das NLS-Peptid gekoppelt war, und ODN 5 erfolgte durch T4 DNA Ligase in Anwesenheit von Eco 31l und wurde durch Erhitzen auf 70°C gestoppt. Das resultierende Gemisch wurde konzentriert und mit Eco 31l und T7 Polymerase in Abwesenheit von Desoxyribonukleotid-Triphosphaten behandelt. Die Aufreinigung erfolgte durch Anionenaustauschchromatographie. Der Nachweis der erfolgreichen Kopplung der Substituenten an die DNA-Expressionskonstrukte erfolgte durch Restriktionsverdau, der die erfolgreiche Kopplung bestätigte.

### Beispiel 6: Stimulation von immunologisch relevanten Oberflächenproteinen durch substituentmodifizierte Trägermoleküle

Eine Funktionsüberprüfung der substituentmodifizierte Trägermoleküle erfolgte mittels eines Stimulationsexperimentes von B-Zellen. Die Inkubation von RPMI-8226 Zellen (Zelllinie der B-Reihe) erfolgte über 48h mit äquimolaren Mengen der eingesetzten Moleküle (1Mikromol). Die Auswertung der Ergebnisse wurde mittels FACS-Messung durchgeführt. Die erhaltenen; Werte sind bezogen auf den unstimulierten Zustand (Kontrolle). Der Oberflächenmarker CD80 wurde als % positive Zellen bestimmt.

## Patentansprüche

1. Nicht-kodierendes Desoxyribonukleinsäuremolekül, welches aus einer teilweise einzelsträngigen, hantelförmigen, kovalent geschlossenen Kette von Desoxyribonukleotidresten besteht und eine oder mehrere Sequenzen der Basenfolge N¹N²CGN³N⁴ im einzel- und / oder doppelsträngigen Bereich enthält, wobei N¹N² ein Element aus der Gruppe GT, GG, GA, AT oder AA, N³N⁴ ein Element aus der Gruppe CT oder TT, sowie C Desoxycytosin, G Desoxyguanosin, A Desoxyadenosin und T Desoxythymidin ist, **dadurch gekennzeichnet, dass** ein oder mehrere Substituenten durch kovalente Bindungen mit dem nicht-kodierenden Desoxyribonukleinsäuremolekül verknüpft sind, wobei wenigstens ein Substituent ein Kernlokalisationssignal gemäß Seq.ID 6 ist.

2. Desoxyribonukleinsäuremolekül nach Anspruch 1, wobei ein zum Kernlokalisationssignal unterschiedlicher Substituent ausgewählt ist aus der Gruppe der Peptide, Proteine, Saccharide, antigenen Strukturen, DNA und/oder RNA.

3. Desoxyribonukleinsäuremolekül nach Anspruch 1 oder *2*, wobei im Falle der kovalenten Bindung von mehr als einem Substituenten, diese aus gleichen oder verschiedenen Gruppen ausgewählt sind.

4. Desoxyribonukleinsäuremolekül nach Anspruch 3, wobei mehrere Substituenten, die aus einer der in Anspruch 2 genannten Gruppe stammen, identisch sind oder sich voneinander unterscheiden.

5. Desoxyribonukleinsäuremolekül nach einem oder mehreren der Ansprüche 1 bis 4, wobei chemisch modifizierte Substituenten und/oder chemisch modifizierte Nukleotide kovalent miteinander verbunden sind.

6. Desoxyribonukleinsäuremolekül nach einem oder mehreren der Ansprüche 1 bis 4, wobei Substituent und Nukleotid über ein bifunktionales Kopplungsreagenz miteinander kovalent verbunden sind.

7. Verfahren zur Herstellung von mit Substituenten gekoppelten nicht- kodierenden Desoxyribonukleinsäuremolekülen nach einem der Ansprüche 1 bis 6, bestehend aus folgenden Verfahrensschritten:
a. Zwei Oligodesoxyribonukleotide, welche partiell selbstkomplementär sind und von denen wenigstens eines chemisch modifiziert ist, werden unter geeigneten Reaktionsbedingungen zumindest mit dem Kernlokalisationsignal gemäß Seq.ID 6 gekoppelt,
b. anschließend wird den aufgereinigten Produkten der Kopplungsreaktion durch kurzes Erhitzen und Abkühlen auf Eis die partielle Basenpaarung sowie das Ausbilden von Haarnadelstrukturen ermöglicht, und
c. die daraus resultierenden selbstkomplementären Überhänge der teilweise doppelsträngigen nicht-kodierenden Nukleinsäuremoleküle werden miteinander oder mit Oligodesoxynukleotiden, welche mit anderen Substituenten kovalent verbunden sind, ligiert,
d. gefolgt von einer Exonukleasebehandlung zum Abbau von nicht kovalent geschlossenen Ketten aus substituentgekoppelten Oligodesoxyribo-nukleotiden, bevor
e. abschließend die nicht-kodierenden, substituentgekoppelten Desoxyribonukleinsäuremoleküle aufgereinigt werden.

8. Verfahren nach Anspruch 7, wobei ein zum Kernlokalisationssignal unterschiedlicher Substituenten durch kovalente Bindung mit dem Desoxyribonukleinsäuremolekül verknüpft wird, wobei dieser Substituent ausgewählt ist aus der Gruppe der Peptide, Proteine, Saccharide, antigenen Strukturen, DNA und/oder RNA.

9. Verfahren nach Anspruch 7, wobei Oligodesoxyribonukleotide zur Kopplung eines Substituenten verwendet werden, die durch eine oder mehrere alkylische Carbonsäure-, Amin-, Thiol- oder Aldehydfunktionen oder eine Maleimidsäu-regruppe chemisch modifiziert sind.

10. Verfahren nach Anspruch 7, wobei Substituenten verwendet werden, die durch eine oder mehrere alkylische Carbonsäure-, Amin-, Thiol- oder Aldehydfunktionen oder eine Maleimidsäuregruppe chemisch modifiziert sind.

11. Verwendung von Desoxyribonukleinsäuremolekülen nach einem der Ansprüche 1 bis 6 zur Herstellung eines Impfstoffes als Adjuvans in der therapeutischen oder prophylaktischen Vakzinierung in Menschen oder höheren Tieren.

12. Verwendung von Desoxyribonukleinsäuremolekülen nach einem der Ansprüche 1 bis 6 zur Herstellung eines Impfstoffes für die *in vitro* oder *in vivo* Immunstimulation in Menschen oder höheren Tieren.

13. Verwendung der Desoxyribonukleinsäuremoleküle nach einem der Ansprüche 1 bis 6 zur Herstellung eines Impfstoffes für die Immunstimulation in Menschen oder höheren Tieren, welcher neben den besagten Desoxyribonukleinsäuremolekülen noch substituentgekoppelte, hantelförmige, linear kovalent geschlossene DNA-Expressionskonstrukten enthält.

14. Verwendung nach Anspruch 13, wobei der Substituent der hantelförmigen, linear kovalent geschlossenen DNA-Expressionskonstrukte ausgewählt ist aus der Gruppe der Peptide, Proteine, Saccharide, antigenen Strukturen, DNA und/oder RNA.

15. Verwendung nach Anspruch 13, wobei die hantelförmigen, linear kovalent geschlossenen DNA-Expressionskonstrukte wenigstens einmal mit einem Kernlokalisationssignal substituiert sind.

16. Impfstoff, umfassend ein nicht-kodierendes Desoxyribonukleinsäuremolekül, welches aus einer teilweise einzelsträngigen, hantelförmigen, kovalent geschlossenen Kette von Desoxyribonukleotidresten besteht und eine oder mehrere Sequenzen der Basenfolge N¹N²CGN³N⁴ im einzel- und / oder doppelsträngigen Bereich enthält, wobei N¹N² ein Element aus der Gruppe GT, GG, GA, AT oder AA, N³N⁴ ein Element aus der Gruppe CT oder TT, sowie C Desoxycytosin, G Desoxyguanosin, A Desoxyadenosin und T Desoxythymidin ist, wobei ein oder mehrere Substituenten durch kovalente Bindungen mit dem nicht-kodierenden Desoxyribonukleinsäuremolekül verknüpft sind, wobei wenigstens ein Substituent ein Kernlokalisationssignal gemäß Seq.ID 6 ist.

17. Impfstoff nach Anspruch 16, welcher zusätzlich substituentengekoppelte, hantelförmige, linear kovalent geschlossene DNA-Expressionskonstrukte umfasst.

## Claims

1. Non-coding deoxyribonucleic acid molecule, which consists of a partially single-stranded, dumbbell-shaped covalently closed chain of deoxyribonucleotide residues and contains one or more sequences of the base sequence N¹N²CGN³N⁴ in its single- and/or double-stranded region, wherein N¹N² is an element chosen from the group comprising GT, GG, GA, AT or AA, N³N⁴ is an element chosen from the group comprising CT or TT, and C is deoxycytosine, G is deoxyguanosine, A is deoxyadenosine and T is deoxythymidine, **characterized in that** one or more substituents are covalently attached to said non-coding deoxyribonucleic acid molecule, wherein at least one substituent is a nuclear localization signal pursuant to Seq.ID 6.

2. Deoxyribonucleic acid molecule according to claim 1, wherein a substituent different from the nuclear localization signal is selected from the group of peptides, proteins, saccharides, antigenic structures, DNA and/or RNA.

3. Deoxyribonucleic acid molecule according to claim 1 or 2, wherein in the case of covalent attachment of more than one substituent, these are selected from the same or different groups.

4. Deoxyribonucleic acid molecule according to claim 3, wherein several substituents originating from one of the groups named in claim 2 are identical or different from each other.

5. Deoxyribonucleic acid molecule according to one or several of the claims 1 to 4, wherein chemically modified substituents and / or chemically modified nucleotides are covalently attached to each other.

6. Deoxyribonucleic acid molecule according to one or several of the claims 1 to 4, wherein the substituent and the nucleotide are covalently attached to each other by means of a bi-functional coupling reagent.

7. Method to manufacture non-coding deoxyribonucleic acid molecules that are attached to substituents according to one of the claims 1 to 6, consisting of the following method steps:
a. two oligodeoxyribonucleotides that are partially self-complementary, and of which at least one is chemically modified, are attached at least to the nuclear localization signal pursuant to Seq.ID 6 under suitable conditions,
b. subsequently, the purified products of the coupling reaction are allowed to form partial base pairs and hairpin structures by brief heating and cooling on ice, and
c. the resulting self-complementary overhangs of the partially double-stranded non-coding nucleic acid molecules are ligated to each other or to oligodeoxynucleotides that are covalently attached to other substituents,
d. followed by exonuclease treatment to degrade non-covalently closed chains of oligodeoxyribonucleotides that are attached to substituents, prior to
e. finally purifying the non-coding deoxyribonucleic acid molecules that are attached to substituents.

8. Method according to claim 7, wherein a substituent different from the nuclear localization signal is covalently attached to the deoxyribonucleic acid molecule, wherein this substituent is selected from the group of peptides, proteins, saccharides, antigenic structures, DNA and/or RNA.

9. Method according to claim 7, wherein oligodeoxyribonucleotides are employed for the attachment of a substituent that are chemically modified by one or several alcylic carboxylic acid-, amine-, thiole-, or aldehyde functional groups or a maleimide group.

10. Method according to claim 7, wherein substituents are employed that are chemically modified by one or several alcylic carboxylic acid-, amine-, thiole-, or aldehyde functional groups or a maleimide group.

11. Use of deoxyribonucleic acid molecules according to one of the claims 1 to 6 for the manufacture of a vaccine as adjuvant in the therapeutic or prophylactic vaccination in humans or higher animals.

12. Use of deoxyribonucleic acid molecules according to one of the claims 1 to 6 for the manufacture of a vaccine for the *in vitro* or *in vivo* immune stimulation in humans or higher animals.

13. Use of deoxyribonucleic acid molecules according to one of the claims 1 to 6 for the manufacture of a vaccine for the immune stimulation in humans or higher animals, which apart from said deoxyribonucleic acid molecules also contains dumbbell-shaped, linear covalently closed DNA expression constructs that are attached to substituents.

14. Use according to claim 13, wherein the substituent of the dumbbell-shaped, linear covalently closed DNA expression constructs is selected from the group of peptides, proteins, saccharides, antigenic structures, DNA and / or RNA.

15. Use according to claim 13, wherein the dumbbell-shaped, linear covalently closed DNA expression constructs are substituted at least once with a nuclear localization signal.

16. Vaccine comprising a non-coding deoxyribonucleic acid molecule, which consists of a partially single-stranded, dumbbell-shaped covalently closed chain of deoxyribonucleotide residues and contains one or more sequences of the base sequence N¹N²CGN³N⁴ in its single- and/or double-stranded region, wherein N¹N² is an element chosen from the group comprising GT, GG, GA, AT or AA, N³N⁴ is an element chosen from the group comprising CT or TT, and C is deoxycytosine, G is deoxyguanosine, A is deoxyadenosine and T is deoxythymidine, wherein one or more substituents are covalently attached to said non-coding deoxyribonucleic acid molecule, wherein at least one substituent is a nuclear localization signal pursuant to Seq.ID 6.

17. Vaccine according to claim 16, which additionally comprises dumbbell-shaped, linear covalently closed DNA expression constructs that are attached to substituents.

## Revendications

1. Molécule d'acide désoxyribonucléique non codante, qui se compose d'une chaîne de résidus de désoxyribonucléotide fermée de manière covalente; en forme de haltère et partiellement à simple brin, , et qui contient une ou plusieurs séquences ayant comme séquence de bases N¹N²CGN³N⁴ dans la région à simple brin et/ou à double brin, où N¹N² est un élément du group GT, GG, GA, AT ou AA, N³N⁴ est un élément du groupe CT ou TT, ainsi que C est désoxycytosine, G est désoxyguanosine, A est désoxyadenosine et T est désoxythymidine, **caractérisé en ce que** un ou plusieurs substituants sont reliés avec la molécule d'acide désoxyribonucléique non codante par une liaison covalente, dans lesquels au moins un substituant est un signal de localisation nucléaire selon Seq.ID 6.

2. Molécule d'acide désoxyribonucléique selon la revendication 1, dans laquelle un substituant différent du signal de localisation nucléaire est choisi du groupe des peptides, des protéines, des saccharides, des structures antigènes, d'ADN et/ou ARN.

3. Molécule d'acide désoxyribonucléique selon la revendication 1 ou 2, dans laquelle au cas d'une liaison covalente avec plus d'un substituant ceux-ci sont choisis de groupes pareils ou divers.

4. Molécule d'acide désoxyribonucléique selon la revendication 3, dans laquelle plusieurs substituants, provenant d'un des groupes nommés dans la revendication 2, sont identiques ou non identiques.

5. Molécule d'acide désoxyribonucléique selon l'une ou plusieurs des revendication 1 à 4, dans laquelle substituants modifiés chimiquement et/ou nucléotides modifiés chimiquement sont reliés d'une manière covalente.

6. Molécule d'acide désoxyribonucléique selon l'une ou plusieurs des revendications 1 à 4, dans laquelle le substituant et le nucléotide le substituant et le nucléotide sont reliés d'une manière covalent par moyen d'un réactif de couplage bifonctionnel.

7. Procédé de fabrication de molécules d'acide désoxyribonucléique non codantes, qui sont couplées à des substituants, selon l'une des revendications 1 à 6, comportant les étapes de procédé suivantes:
a. Deux oligodésoxyribonucléotides, qui sont partiellement auto-complémentaires et dont au moins un est modifié chimiquement, sont couplés, dans des conditions appropriées, au moins au signal de localisation nucléaire selon Seq.ID 6,
b. Ensuite il est permis aux produits de la réaction de couplage purifiés de former d'une manière partielle des paires de base et de former des boucles en épingle à cheveux, par moyen de réchauffement bref et refroidissement sur glace,
c. Les saillies auto-complémentaires, en résultant, des molécules d'acide désoxyribonucléique non codantes et partiellement à double brin sont reliées d'une manière covalente l'une à l'autre ou à des oligodésoxynucléotides qui sont reliés à d'autres substituants d'une manière covalente,
d. Suivi d'un traitement d'exonucléase pour la dégradation de chaînes d'oligodésoxyribonucléotides, couplés à des substituants, non fermées d'une manière covalente, avant que
e. Les molécules d'acide désoxyribonucléique non codantes couplées à des substituants sont finalement purifiées.

8. Procédé selon la revendication 7, dans lequel un substituant différent du signal de localisation nucléaire est relié à une molécule d'acide désoxyribonucléique par liaison covalente, dans lequel ce substituant est choisi du groupe des peptides, protéines, saccharides, structures antigènes, ADN et/ou ARN.

9. Procédé selon la revendication 7, dans lequel des oligodésoxyribonucléotides sont employés au couplage d'un substituant qui sont modifiés chimiquement par une ou plusieurs fonctions d'acide carboxylique d'alkyle, amines, thiols ou aldéhydes ou par un groupe d'acide maléimide.

10. Procédé selon la revendication 7, dans lequel des substituants sont employés qui sont modifiés chimiquement par une ou plusieurs fonctions d'acide carboxylique d'alkyle, amines, thiols ou aldéhydes ou un groupe d'acide maléimide.

11. Utilisation de molécules d'acide désoxyribonucléique selon l'une des revendications 1 à 6 pour la fabrication d'un vaccin comme adjuvant dans la vaccination thérapeutique ou prophylactique chez les hommes ou les animaux supérieurs.

12. Utilisation de molécules d'acide désoxyribonucléique selon l'une des revendications 1 à 6 pour la fabrication d'un vaccin pour immunostimulation in vitro ou in vivo chez les hommes ou les animaux supérieurs.

13. Utilisation de molécules d'acide désoxyribonucléique selon l'une des revendications 1 à 6 pour la fabrication d'un vaccin pour la immunostimulation dans des humains ou animaux supérieurs qui contient, à part desdites molécules d'acide désoxyribonucléique, encore des construits d'expression d'ADN linéairement fermés d'une manière covalente, en forme d'haltère et couplés à des substituants.

14. Utilisation selon la revendication 13, dans laquelle le substituant des construits d'expression d'ADN linéairement fermés d'une manière covalente et en forme d'haltère est choisi du groupe des peptides, protéines, saccharides, structures antigénes, ADN et/ou ARN.

15. Utilisation selon la revendication 13, dans laquelle les construits d'expression d'ADN linéairement fermés d'une manière covalente et en forme d'haltère sont au moins une fois substitués à un signal de localisation nucléaire.

16. Vaccin, comprenant une molécule d'acide désoxyribonucléique non codante qui est composée d'une chaîne de résidus de désoxyribonucléotide fermée de manière covalente, en forme de haltère et partiellement à simple brin, et contient une ou plusieurs séquences de la séquence de base N¹N²CGN³N⁴ dans la région à simple brin et/ou à double brin, dans laquelle N¹N² est un élément du groupe GT, GG, GA, AT ou AA, N³N⁴ est un élément du groupe CT ou TT, ainsi que C est désoxycytosine, G est désoxyguanosine, A est désoxyadenosine et T est désoxythymidine, dans laquelle un ou plusieurs substituants sont reliés avec la molécule d'acide désoxyribonucléique non codante par une liaison covalente, dans lesquels au moins un substituant est un signal de localisation nucléaire selon Seq.ID 6.

17. Vaccin selon la revendication 16, qui contient encore des construits d'expression d'ADN linéairement fermés d'une manière covalente qui sont en forme d'haltère et couplés à des substituants.
